# EUROPEAN PATENT APPLICATION

(11) **EP 4 520 341 A1**
(43) Date of publication of application: **12.03.2025**
(21) Application number: 23799705.1
(22) Date of filing: 04.05.2023
(51) Int. Cl.: A61K 35/28, A61P 17/02, C12N 5/0775

(54) **COMPOSITION FOR WOUND TREATMENT COMPRISING STEM CELL-DERIVED EXTRACELLULAR VESICLES WITH ENHANCED EFFICACY**

(30) Priority: 04.05.2022 KR 20220055643
(71) Applicant: S&E Bio Co., Ltd., Seoul 06351 (KR); Samsung Life Public Welfare Foundation, Seoul 04348 (KR)
(72) Inventor: KIM, Eun Hee, Seoul 06608 (KR); SUNG, Ji Hee, Seoul 08716 (KR); SHIN, Eun Kyoung, Seoul 05807 (KR); BANG, Oh Young, Seoul 06351 (KR); KIM, Ji Eun, Seoul 06351 (KR)
(74) Representative: Cabinet Becker et Associés
(86) International application number: PCT/KR2023/006122
(87) International publication number: WO 2023/214824

(57) **Abstract**

The present invention relates to a composition for for use in wound healing or wound recovery comprising extracellular vesicles derived from a three-dimensional spheroid-type cell aggregate produced using a novel production method, and a method for wound healing. The extracellular vesicles produced by the novel method of the present invention can rapidly promote wound recovery and thus can be used as various medicines, cosmetics, and foods for wound healing, regeneration, and recovery.

## Description

### [Technical Field]

The present invention relates to a composition for wound healing including extracellular vesicles derived from a three-dimensional spheroid-type cell aggregate that are produced using a novel production method.

### [Background Art]

Treatments using stem cells, particularly mesenchymal stem cells (MSCs), and positive clinical results have been reported for various diseases. However, stem cell therapy has a risk of cell-related side effects such as vascular occlusion, tumor formation, and coagulation disorders, and efficacy verification through clinical trials is still required. It is known that a paracrine effect of stem cells induces the promotion of the regeneration and vascular regeneration of surrounding skin cells, and in particular, extracellular vesicles (EV) are known as a major efficacious factor in the paracrine effect.

The extracellular vesicles are classified into exosomes and macrovesicles depending on a size, and the exosomes have a diameter of 30 to 150 nm, and the microvesicles have a size of 100 to 1,000 nm. The extracellular vesicles are parts of the cell membrane that have been separated into the blood, and are known to mediate intercellular communication by containing both protein and nuclear components. Using extracellular vesicles instead of stem cells not only increases safety by minimizing the side effects by using stem cells, but also is advantageous in terms of biodistribution and production process.

However, mass production, obtaining method, and the like for using extracellular vesicles derived from stem cells have not yet been established, and much research has been not conducted on methods to further enhance the efficacy of extracellular vesicles while maintaining the characteristics of stem cell-derived extracellular vesicles.

Accordingly, there is a need for extracellular vesicles with improved efficacy and novel therapeutic agents using the same.

### [Disclosure]

### [Technical Problem]

Therefore, the present inventors studied a method capable of shortening a culture time while enabling mass production, in order to produce three-dimensional spheroid-type cell aggregates or extracellular vesicles derived therefrom. As a result, the present inventors confirmed that extracellular vesicles with improved wound regeneration and wound recovery ability were produced by producing a three-dimensional spheroid-type cell aggregate (3D-static-spheroid) through static culture using a microwell and producing extracellular vesicles using the same, and then completed the present invention.

Accordingly, an object of the present invention is to prepare extracellular vesicles derived from a three-dimensional spheroid-type cell aggregate with improved wound healing ability and to provide a composition for wound healing, regeneration or recovery including the same.

### [Technical Solution]

An aspect of the present invention provides a pharmaceutical composition for use in wound healing or wound recovery including extracellular vesicles derived from a three-dimensional spheroid-type cell aggregate prepared by (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

Another aspect of the present invention provides a cosmetic composition for use in wound healing or wound recovery including extracellular vesicles derived from a three-dimensional spheroid-type cell aggregate prepared by (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

Yet another aspect of the present invention provides a quasi-drug composition for use in wound healing or wound recovery including extracellular vesicles derived from a three-dimensional spheroid-type cell aggregate prepared by (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

Still another aspect of the present invention provides a method for producing a composition for wound healing or wound recovery including extracellular vesicles derived from a three-dimensional spheroid-type cell aggregate including: (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

Still another aspect of the present invention provides a method for wound healing including (a) preparing a three-dimensional spheroid-type cell aggregate by 3-dimensionally (3D) culturing stem cells in a microwell with a diameter of 200 to 800 µm and a depth of 100 to 1000 µm; (b) preparing extracellular vesicles derived from the three-dimensional spheroid-type cell aggregate by isolating the extracellular vesicles from the three-dimensional spheroid-type cell aggregate; and (c) treating the extracellular vesicles derived from the three-dimensional spheroid-type cell aggregate prepared in step (b) to a subject in need thereof.

### [Advantageous Effects]

According to the present invention, the extracellular vesicles produced by the novel method can rapidly promote wound recovery and thus can be used as various medicines, cosmetics, and foods for wound healing, regeneration, and recovery.

### [Description of Drawings]

FIG. 1 is a schematic diagram illustrating a process of producing a 3D spheroid-type cell aggregate and isolating extracellular vesicles therefrom.
FIG. 2A is a diagram of observing a microwell containing a 3D-dynamic-PEG spheroid culture medium.
FIG. 2B is a diagram of observing a microwell containing a 3D-static-spheroid culture medium.
FIG. 2C is a diagram illustrating changes in aggregate area according to culture periods of 3D-dynamic-PEG spheroid and 3D-static-spheroid produced using a microwell.
FIG. 3 is a diagram of comparing size distributions of 3D-static-spheroid and 3D-dynamic-PEG spheroid.
FIG. 4 is a diagram of observing the shape of 3D-static-spheroid EV with an electron microscope.
FIG. 5 is a diagram illustrating results of nanoparticle tracking analysis (NTA) to confirm the concentration and size distribution of 3D-static-spheroid EV.
FIG. 6 is a diagram illustrating results of ELISA and Western blot to confirm expression markers of 3D-static-spheroid EV.
FIG. 7 is a diagram of comparing productions of 3D-static-spheroid EV, 3D-dynamic-PEG spheroid EV, and 2D-EV per derived cell.
FIG. 8 is a diagram illustrating miRNAs and proteins highly expressed in 3D-static-spheroid EV compared to 2D-EV.
FIG. 9 is a diagram illustrating miRNAs and proteins highly expressed in 3D-static-spheroid EV compared to 3D-dynamic-PEG spheroid EV.
FIG. 10A is a diagram illustrating a result of confirming differences in EV production, EV size, and EV-containing protein amount depending on a donor in 2D-EV and 3D-static-spheroid EV (WJ-3D EV).
FIG. 10B is a diagram illustrating a result of confirming donor variation and differences in expression levels of miRNAs related to angiogenesis and nerve regeneration in 2D cultured WJ-MSC, 3D cultured WJ-MSC, 2D-EV, and 3D-static-spheroid EV.
FIG. 11 is a diagram illustrating changes in mRNA expression, after treating 3D-static-spheroid EV or 2D-EV to human umbilical vascular endothelial cells (HUVECs) or primitive neural stem cells (pNSCs) as neural stem cells.
FIG. 12 is a diagram illustrating a result of confirming wound recovery information for 14 days after treatment with 3D-static-spheroid EV (3D-EV), WJ-MSCs, and PBS.
FIG. 13 is a graph showing quantifying the recovery of wound areas in 3D-static-spheroid EV and PBS (Control) treated groups.
FIG. 14 is a diagram illustrating a result of confirming an increase in epidermal thickness by 3D-static-spheroid EV treatment through staining of the cross-sectional area of wounded skin.
FIG. 15 is a diagram illustrating a result of confirming the recovery of wound areas through staining after 14 days in 3D-static-spheroid EV (EV) and PBS (Control) treated groups.
FIG. 16 is a graph showing quantifying an effect of 3D-static-spheroid EV treatment on an increase in thickness of the epidermis and the epidermal periphery.
FIG. 17 is a schematic diagram illustrating a wound recovery experimental protocol using a chamber model.
FIG. 18 is a diagram illustrating results of confirming the wound recovery areas after removing chambers in 3D-static-spheroid EV (EV) and PBS groups.
FIG. 19 is a graph showing confirming and quantifying wound areas after removing chambers in 3D-static-spheroid EV (EV) and PBS groups.
FIG. 20 is a diagram illustrating results of confirming an increase in epidermal thickness according to 3D-static-spheroid EV treatment in a chamber model.
FIG. 21 is a diagram illustrating ELISA results of confirming expression changes of angiogenesis-related factors VEGF, angiopoietin-1 (Angpt-1), and angiopoietin-2 (Angpt-2) (FIG. 21A), inflammation-related factors IL-1b, TNF-a, and IL-6 (FIG. 21B), and an anti-inflammatory factor IL-10 (FIG. 21C) after 7 or 14 days of removing chambers in chamber models.
FIG. 22A is a diagram illustrating a result of confirming mobility of fibroblasts after treatment with 3D spheroid EV (EV) and FIG. 22B is a diagram illustrating a result of confirming mobility of keratinocytes.
FIG. 23 is a graph showing a result of comparing increased expression amounts of VEGF, Hif-1a, and FGF related to angiogenesis by treating HUVEC cells with 3D spheroid EV and 2D-EV.
FIG. 24 is a diagram illustrating a result of comparing effects of vascular tube formation by treating cells with 3D spheroid EV and 2D-EV.
FIG. 25 is a diagram illustrating a result of confirming the size, roundness, and solidity of 3D spheroids produced after varying the diameter and depth of a microwell and the number of cells per well in the production of 3D-static spheroid EV.
FIG. 26 is a diagram illustrating a result of comparing the expression levels of miRNA132 and miRNA210 expressed in 3D-static-spheroid EV and 2D-EV produced under various conditions.
FIG. 27 is a diagram illustrating a result of confirming angiogenesis ability of 3D-static-spheroid EV and 2D-EV produced under various conditions through a tube formation experiment.

### [Best Mode for the Invention]

The present invention provides a pharmaceutical composition for wound healing or wound recovery including extracellular vesicles derived from a three-dimensional spheroid-type cell aggregate prepared by (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

In the present invention, the cells may be used without limitation as long as the cells are cells capable of isolating extracellular vesicles, and may be cells isolated from a natural organism. In addition, the cells may be derived from any type of animal or plant, including humans and non-human mammals, and may be various types of immune cells, tumor cells, and stem cells, and preferably, the stem cells may be mesenchymal stem cells, pluripotent stem cells, induced pluripotent stem cells, or embryonic stem cells.

In the present invention, the 3D culture means culturing in a three-dimensional arrangement in vitro, and unlike 2D culture, the cell growth in the 3D culture may grow cells in all directions in vitro and may be more similar to an in vivo cell environment.

In the present invention, the 3D culture in step (a) may be performed by any 3D cell culture technique known in the art to which the present invention pertains, and may be a cell culture using, for example, microwell array culture, porous microsphere culture, hanging drop culture, low attachment plate culture, membrane-based cell-detachment culture, thermal lifting culture, centrifugation culture, semisolid medium culture, and the like. Preferably, the 3D culture may be dynamic culture or static culture, more preferably static culture. In the present invention, when the 3D culture of step (a) is static culture, it makes it easier to culture without requiring devices necessary for shaking culture, and mass-culture is enabled in the Good Manufacturing Practices (GMP) manufacturing place.

In the present invention, the 3D culture in step (a) may be performed for 1 to 10 days, preferably 2 to 4 days. Particularly, in the present invention, when the culture in step (a) is performed for 2 to 4 days, the viability of cells present in the three-dimensional spheroid-type cell aggregate is maintained at a high level, and compared to a conventional process of producing a three-dimensional spheroid-type cell aggregate, the culture time is relatively short, so that it is possible to rapidly produce a three-dimensional spheroid-type cell aggregate and extracellular vesicles derived therefrom.

In the present invention, the 3D culture in step (a) may be performed by dispensing mesenchymal stem cells in a microwell at a density of 100 to 1000 cells/well, preferably a density of 100 to 600 cells/well, and more preferably a density of 100 to 500 cells/well.

In the present invention, the isolating of the extracellular vesicles in step (b) may be performed by physical isolation or chemical isolation. The physical isolation may be extrusion of a sample containing cells or a cell aggregate, and the chemical isolation may be treatment with a chemical substance capable of isolating extracellular vesicles from cells or a cell aggregate. For example, the isolating of the present invention may be performed by using a method selected from the group consists of sonication, cell lysis, homogenization, freeze-thawing, electroporation, chemical treatment, mechanical degradation, and treatment of physical stimuli externally applied to cells. An ion or affinity chromatography method may be a physiochemical method of isolation through binding of ions to specific biomarkers or an immunoaffinity EV capture from hansabiomed life sciences, an exosome purification reagent from creative biolabs, and an ExoCAS-2 reagent or method from microgentas, which destroys cells and binds and isolates only extracellular vesicles through polymers and reagents, preferably a tangential flow filtration (TFF) method, but is not limited thereto.

In the present invention, MicroRNA is named by attaching "mir" in the front and adding "-" and a number at the end. At this time, the numbers often indicate the order to be named, and for example, mir-123 was named before mir-156 and is expected to have been identified earlier. "mir-" indicates pre-microRNA, and capitalized "miR-" refers to mature microRNA. Except for one or two sequences, microRNAs with almost identical sequences are named by adding small letters. For example, miR-121a and miR-121b were generated from respective precursors mir-121a and mir-121b, and their sequences are very similar. Mature microRNAs are identical, but pre-microRNA located at different sites on a genome are named by adding "-" and a number. For example, the pre-microRNAs mir-121-1 and mir-121-2 become the same mature microRNA (miR-121), but are located at different sites on the genome. Species-specific names of microRNAs are indicated in the front. For example, hsa-miR-123 is Homo sapiens microRNA, and oar-miR-123 is Ovis aries microRNA. 'v' means viral (miRNA encoded by a viral genome) and 'd' means Drosophila microRNA. When two mature microRNAs are derived from different arms (3' arm or 5' arm) of the same pre-microRNA, the mature microRNAs are named by adding '-3p' or '-5p' to the end. miR-142-3p is derived from the 3' arm, and miR-142-5p is derived from the 5' arm. The nomenclature of MicroRNA generally follows the above criteria, but there are exceptions.

In the present invention, the extracellular vesicles may highly express various substances exhibiting wound healing efficacy compared to known extracellular vesicles. For example, preferably, the extracellular vesicles of the present invention may highly express at least one selected from the group consisting of miR-146a, miR-27a, miR-132, miR-184, miR-210, and miR-301b compared to extracellular vesicles derived from a spheroid-type cell aggregate obtained by 3D dynamic culture of mesenchymal stem cells; or highly express at least one selected from the group consisting of miR-27a, miR-146a, and miR-146b compared to extracellular vesicles derived from 2D-cultured mesenchymal stem cells, and may highly express a vascular endothelial growth factor (VEGF), a hypoxia-inducible factor 1-alpha (Hif-1a) and a fibroblast growth factor (FGF).

In addition, the extracellular vesicles may be incorporated and internalized into cells upon treatment with cells, and when incorporated and internalized into the cells, a clinically significant substance highly expressed in the extracellular vesicles may be effectively delivered to the cells to be highly expressed in the cells.

In the present invention, the extracellular vesicles derived from the three-dimensional spheroid-type cell aggregates that exhibit wound healing, regeneration, and recovery effects may be used interchangeably with "3D-static-spheroid-EVs". In addition, in comparison, the extracellular vesicles derived from the 3D dynamically cultured spheroid-type cell aggregate may be used interchangeably with "3D-dynamic-PEG-spheroid-EVs".

In the present invention, the "extracellular vesicles derived from the spheroid-type cell aggregate obtained by 3D dynamic culture of mesenchymal stem cells" are any extracellular vesicles isolated from a spheroid-type cell aggregate obtained by 3D dynamic culture of mesenchymal stem cells without limitation, preferably extracellular vesicles disclosed in Registration Patent (application No. 10-2016-0053026, method for producing stem cell-derived extracellular vesicles).

In the present invention, the three-dimensional spheroid-type cell aggregate may have an average diameter of 74.43 ± 7.756 µm, preferably a size range of 55 to 95.0 µm. As a result of measuring the size distribution of 155 three-dimensional spheroid-type cell aggregates, the average diameter may be 74.43 µm and the coefficient of variance (CV) may be 9.59%. The three-dimensional spheroid-type cell aggregate of the present invention may have a higher kurtosis in size distribution than the "spheroid-type cell aggregate obtained by 3D dynamic culture of mesenchymal stem cells". Therefore, the size of the three-dimensional spheroid-type cell aggregate is smaller than that of the "spheroid-type cell aggregate obtained by 3D dynamic culture of mesenchymal stem cells" and may have a relatively uniform size distribution.

In the present invention, the microwell may be coated with at least one selected from the group consisting of TMSPMA (3-(Trimetoxysily) propylmethacrylate), HEA (Hydroxyethyl acrylate), GMA (Glycidyl methacrylate), EGDMA (diethyleneglycol dimethacrylate), THFA (Tetrahydrofurfuryl acrylate), HMAA (Hydroxymethul acrylamide), PEA (Phenyl epoxyacrylate), HOFHA (6-Hydroxy-2, 2,3,3,4,4,5,5-octafluoro), EOPT (Polyethoxylated(4)pentaerythritoltetraacrylate), HPA (Hydroxypropyl acrylate), BMA (Buthylmethacrlate), PETIA (Pentaerythritol triacrylate), HDDA (Hexan diol diacrylate), EGPEA (Ethyleneglycol phenyletheracrylate), BM (Benzylmethacrylate), HPPA (Hydroxyphenoxypropyl acrylate), BHPEA (2-(4-Benzoyl-3-hydroxyphenoxy)ethylacrylate), HEMA (Hydroxyethyl methacrylate), HPMA (N-(2-Hydroxypropyl) methacrylamide) and MPC (2-Methacryloyloxyethyl Phosphorylcholine Polymer), but is not limited thereto.

The microwell of the present invention may have a diameter of 200 to 800 µm, preferably 300 to 800 µm, and more preferably 400 to 800 µm.

In addition, the microwell may be a microwell having a flat structure without a depth, or in the case of a microwell with a depth, the microwell may have a structure of 100 to 1000 µm, preferably 100 to 900 µm, and more preferably 200 to 900 µm.

The mesenchymal stem cells cultured by the structure may maintain a high survival rate even after the culture time has elapsed. Preferably, the production yield of cell aggregates may be increased by manufacturing a microarray including 1,000 to 100,000 microwells.

When the extracellular vesicles are produced by the "method for producing the extracellular vesicles derived from the three-dimensional spheroid-type cell aggregate" of the present invention, in addition to the manufacturing advantages of static culture, extracellular vesicles with improved wound healing ability may be mass-produced quickly and efficiently. In particular, the method for producing the extracellular vesicles of the present invention is suitable for GMP applications.

In the present invention, the extracellular vesicles derived from the three-dimensional spheroid-type cell aggregate may induce rapid wound recovery upon treatment to the wound, and induce rapid regeneration and recovery of the wound area by promoting the mobility of fibroblasts and/or keratinocytes in the wound area in the early stage of wound occurrence and promoting angiogenesis.

In the present invention, the angiogenesis refers to the formation of new blood vessels, and means a process of inducing or increasing the mobility of vascular endothelial cells and promoting tube formation in the vascular endothelial cells to form new blood vessels from existing blood vessels.

The wound applied with the extracellular vesicles derived from the three-dimensional spheroid-type cell aggregate of the present invention may include all wound types occurring on the skin without limitation, and may be applied to various skin wounds, such as burns, bedsores, and cuts. The burns are damage to skin cells caused by fire or heat, and the bedsores are chronic ulcers that occur when tissue dies due to inflammation or tissue necrosis caused by poor blood circulation. The cuts mean damage to skin tissue caused by external pressure, and may include, for example, abrasion, bruise, laceration, cut by a blade, stab, cleaver wound, gunshot wound, explosional wound, and cross-cutting wound.

The pharmaceutical composition of the present invention may further include suitable carriers, excipients, and diluents which are commonly used in the preparation of the pharmaceutical composition in addition to the active ingredient. The pharmaceutical composition of the present invention may further include other pharmaceutically active ingredients or active mixtures.

The pharmaceutical composition of the present invention may be formulated and used in the form of oral formulations, such as patches, coverings, powders, granules, tablets, capsules, suspensions, emulsions, syrups, aerosols, etc., external preparations, suppositories, and sterile injectable solutions according to conventional methods, respectively. The carrier, the excipient, and the diluent that may be included in the composition may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil. When the pharmaceutical composition is formulated, the formulation may be prepared by using diluents or excipients, such as a filler, an extender, a binder, a wetting agent, a disintegrating agent, and a surfactant, which are generally used. Solid formulations for oral administration include tablets, pills, powders, granules, capsules, and the like, and these solid formulations may be prepared by mixing at least one or more excipients, for example, starch, calcium carbonate, sucrose or lactose, gelatin, and the like. Further, lubricants such as magnesium stearate and talc are used in addition to simple excipients. Liquid formulations for oral administration may correspond to suspensions, oral liquids, emulsions, syrups, and the like, and may include various excipients, for example, a wetting agent, a sweetener, an aromatic agent, a preservative, and the like, in addition to water and liquid paraffin which are commonly used as simple diluents. Formulations for parenteral administration include sterilized aqueous solutions, non-aqueous solvents, suspensions, emulsions, lyophilized agents, and suppositories. As the non-aqueous solution and the suspension, propylene glycol, polyethylene glycol, vegetable oil such as olive oil, injectable ester such as ethyl oleate, and the like may be used. As a base compound of the suppository, witepsol, macrogol, tween 61, cacao butter, laurinum, glycerogelatin, and the like may be used.

A preferable dose of the pharmaceutical composition of the present invention varies according to the condition and weight of a patient, the degree of a disease, a drug form, and the route and period of administration, but may be properly selected by those skilled in the art. The administration may be performed once a day or several times a day. The dose does not limit the scope of the present invention in any aspect.

The pharmaceutical composition of the present invention may be administered to mammals such as mice, rats, livestock, and humans through various routes. All methods of administration may be expected and for example, the pharmaceutical composition may be administered by oral, percutaneous, rectal or intravenous, intramuscular, subcutaneous, and topical injection.

The definitions of terms for the excipients, binders, disintegrants, lubricants, flavors enhancers, flavoring agents, and the like of the present invention are described in literatures known in the art and include those with the same or similar functions.

Further, the present invention provides a method for wound healing including (a) preparing a three-dimensional spheroid-type cell aggregate by 3-dimensionally (3D) culturing stem cells in a microwell with a diameter of 200 to 800 µm and a depth of 100 to 1000 µm; (b) preparing extracellular vesicles derived from the three-dimensional spheroid-type cell aggregate by isolating extracellular vesicles from the three-dimensional spheroid-type cell aggregate; and (c) treating the extracellular vesicles derived from the three-dimensional spheroid-type cell aggregate prepared in step (b) to a subject in need thereof.

The subject may be mammals, including humans, and may include a patient in need of wound healing, or a patient receiving or needing treatment for wound healing. The extracellular vesicles derived from the three-dimensional spheroid-type cell aggregate may be treated in combination with other drugs or treatment methods, and when treating in combination with other drugs or treatment methods, the extracellular vesicles may be treated simultaneously or sequentially with other drugs or treatment methods.

Further, the present invention provides a cosmetic composition and a quasi-drug composition for wound healing or wound recovery including extracellular vesicles derived from a three-dimensional spheroid-type cell aggregate produced by (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

In this specification, the "cosmetic composition" means a composition composed for the purpose of preparing cosmetics, and may be broadly interpreted to include an external preparation composition for an external preparation. The cosmetic composition according to the present invention may be prepared in any formulation commonly manufactured in the art. For example, the cosmetic composition may have formulations, such as a toner such as a soft toner or a nourishing toner, a spray-type toner, an emulsion such as a facial lotion and a body lotion, a cream such as a nourishing cream, a moisturizing cream, an eye cream, a stick, an essence, a cosmetic ointment, a spray, a gel, a pack, a sunscreen, a makeup base, a foundation such as a liquid type or a spray type, a powder, a makeup remover such as a cleansing lotion and a cleansing oil, a cleansing foam, a soap, a body wash, etc., but is not limited thereto.

The cosmetic composition of the present invention may be used according to a conventional usage method, and the number of used times may vary depending on the skin condition or preference of users.

In this specification, the "quasi-drug" means a product that exhibits the effect of treating, alleviating, handling or preventing diseases, but has a milder effect on the human body than medicine. The quasi-drug includes a product according to classification criteria separately established by the Ministry of Health and Welfare, while excluding products used for the purpose of medicine according to the Pharmaceutical Affairs Act. Specifically, the quasi-drug may be a skin external preparation or a personal hygiene product, but is not limited thereto.

When the composition of the present invention is added to the quasi-drug composition for wound recovery or regeneration, the composition may be added as it is or used together with other quasi-drug ingredients, and may be used appropriately according to a conventional method. The mixing amount of the active ingredients may be appropriately determined depending on the purpose of use. The skin external preparation is not particularly limited thereto, but may be prepared and used in the form of, for example, ointments, lotions, sprays, patches, creams, powders, suspensions, gelling agents, or gels.

The cosmetic composition or quasi-drug composition may be equally cited in the same manner as the description of the pharmaceutical composition.

Further, the present invention provides a method for producing a composition for wound healing or wound recovery including extracellular vesicles derived from a three-dimensional spheroid-type cell aggregate, including (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

According to the producing method, the extracellular vesicles derived from the three-dimensional spheroid-type cell aggregate, which have an excellent wound healing effect, may be quickly mass-produced in accordance with GMP standards.

Duplicated contents are omitted in consideration of the complexity of the present specification, and terms not defined otherwise in the present specification have the meanings commonly used in the art to which the present invention pertains.

Hereinafter, the present invention will be described in detail by Examples. However, the following Examples are just illustrative of the present invention, and the contents of the present invention are not limited to the following Examples.

### [Modes for the Invention]

### Western blot

Cells and extracellular vesicles were dissolved in a radioimmunoprecipitation assay (RIPA) buffer (25 mM Tris-HCl, pH 7.6, 150 mM NaCl, 0.5% triton X-100, 1% Na-deoxycholate, 0.1% sodium dodecyl sulfate (SDS) and protease inhibitor). A total of 20 µg of proteins was isolated by SDS-polyacrylamide gel electrophoresis and transferred to a nitrocellulose membrane (Bio-Rad, Hercules, CA, USA). Thereafter, the nitrocellulose membrane was cultured overnight at 4°C together with a primary antibody against histone H2A.Z, histone H3, lamin A/C, flotillin-1 (1:1,000, Cell signaling technology, Beverly, MA, USA) or calreticulin (1:1,000, ThermoFisher Scientific, Inc., Rockford, IL, USA). After washed with Tris-buffered saline-Tween 20, the nitrocellulose membrane was cultured for 2 hours together with a horseradish peroxidase (HRP)-conjugated secondary antibody (1:1,000, anti-rabbit, Cell Signaling Technology, Beverly, MA, USA). The proteins were detected using a chemiluminescent substrate from ThermoFisher Scientific, Inc. (Waltham, MA, USA). Labeled proteins were visualized via an X-ray film (Agfa, Mortsel, Belgium).

### ELISA

ELISA was performed with a commercial kit according to the manual of an individual manufacturer. The following ELISA kits were used: gentamicin (5111GEN, EuroProxima, Arnhem, Nederland), bovine albumin (8100, Alpha Diagnostic, San Antonio, TX, USA), Hsp70 (Abcam, Cambridge, UK), CD63, CD9 and CD81 (System Biosciences, Palo Alto, CA, USA), histone H2A.Z. (Mybiosource, San Diego, CA, USA), calreticulin (Mybiosource) and cytochrome C (ThermoFisher Scientific, Inc.). All the kits included standard proteins; therefore, the amounts of proteins and extracellular vesicles were determined based on a standard curve of each kit.

### qPCR

RNA was extracted from EVs using Trizol^{™} according to the instructions of the manufacturer and RNA was quantified by a nanodrop. RNA was made into cDNA through a reverse transcription (RT) process, and real-time PCR was performed according to the manual of the manufacturer using Taqman probes suitable for each miRNA and mRNA.

### EV labeling and uptake by cells

Purified EVs were stained with CFSE (5-(and-6)-Carboxyfluorescein Diacetate, Succinimidyl Ester) labeling dye (c-1157, Invitrogen) according to the manufacturer's instructions. Excess dye was removed by ultracentrifugation at 100.000 g for 1 hour. A human NSC cell line (ReNcells^{®}) was treated with the labeled EVs (0.4 µg/ml) and cultured for 24 hours. After treatment, cells were washed twice with PBS and stained using a conventional immunocytochemistry protocol. The cells were incubated overnight at 4°C with a mouse anti-SMA (1:100, Sigma aldrich) antibody. Thereafter, the cells were washed with PBS and cultured with a secondary antibody, DyLight-labeled anti-mouse IgG (1:200, 594 nm, Abcam) antibody. The cells were nuclear-stained using Vectashield^{™} with 1.5 µg/mL 4'-6' diamidino-2-phenylindole (DAPI) (Vector Laboratories), and then the cells were imaged using a confocal microscope (LSM 700, Carl Zeiss, Germany).

### Example 1. Isolation of extracellular vesicles through 3D culture of mesenchymal stem cells

### 1.1 Preparation of mesenchymal stem cells

Wharton's Jelly-derived mesenchymal stem cells (hereinafter, WJ-MSC, Samsung medical center, Seoul, Korea) at passage 5 stage were received and cultured in a 37°C, 5% CO₂ incubator. A growth medium was used with an α-modified eagle's medium (α-MEM, GIBCO, NY, USA) containing 10% fetal bovine serum (FBS) (GIBCO, NY, USA) and 50 µg/mL gentamicin (GIBCO, NY, USA). WJ-MSCs of passage 6 stage were used to prepare a 3D spheroid-type cell aggregate.

### 1.2 3D Production of 3D spheroid-type cell aggregate culture medium

The WJ-MSCs prepared in Example 1.1 were washed with PBS, treated with trypsin (TrypLE^{™} Express, GIBCO, NY, USA) and reacted in a CO₂ incubator for 5 minutes. Thereafter, a new serum-free medium was added to neutralize trypsin and the cells were restored to obtain a cell pellet using a centrifuge. Then, a new serum-free medium was added to prepare a cell suspension, and the cells were counted. After cell counting, 60 ml of the cell suspension was uniformly dispensed in a microarray including microwells coated with 2-methacryloyloxyethyl phosphorylcholine polymer (MPC) and having a diameter and depth of 500 µm × 200 µm to be a density of 400 cells/well, respectively, and maintained in a static state to induce the spontaneous formation of spheroid-type cell aggregates, and cultured in a CO₂ incubator at 37°C for a total of 4 days to prepare a 3D spheroid-type cell aggregate culture medium (hereinafter, referred to as a 3D-static-spheroid culture medium).

### 1.3 Isolation of extracellular vesicles derived from 3D spheroid-type cell aggregate

The 3D-static-spheroid culture medium prepared in Example 1.2 was collected, centrifuged at 2,500 g for 10 minutes, and filtered with a 0.22 µm syringe filter to remove cell debris. Thereafter, the 3D-static-spheroid culture medium passed through 300 kDa of a hollow fiber membrane (Pall, NY, USA) using a tangential flow filtration (TFF) system to remove proteins, and extracellular vesicles were first isolated and purified once more with physiological saline to obtain high-purity 3D-static-spheroid-derived extracellular vesicles (hereinafter, 3D-static-spheroid EVs) of the present invention.

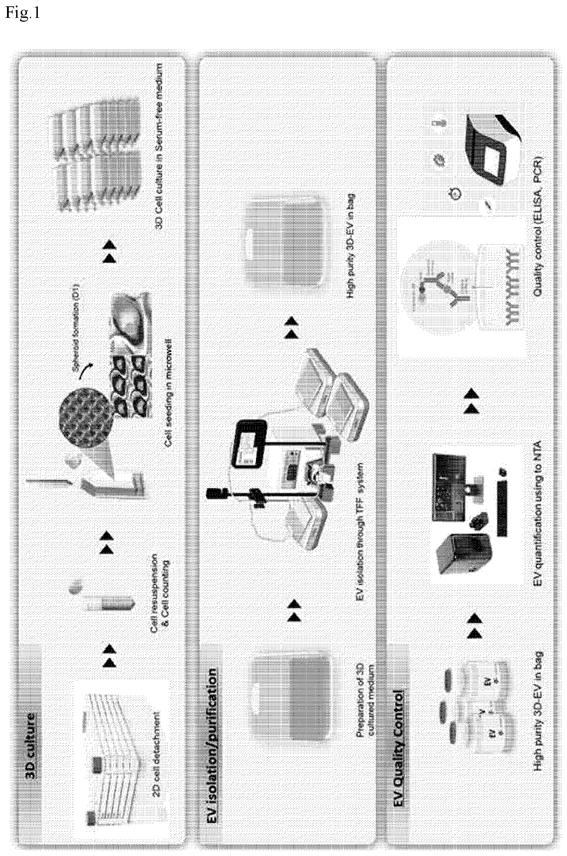

The processes of Examples 1.1 to 1.3 were schematically illustrated in FIG. 1.

### Example 2. Analysis of characteristics of 3D spheroid-type cell aggregate

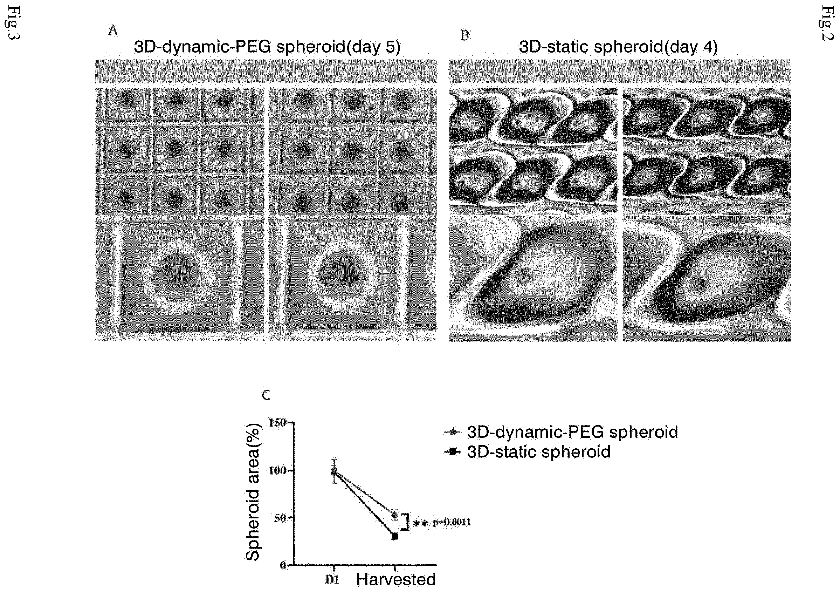

The characteristics of the 3D-spheroid-type cell aggregate (hereinafter, referred to as 3D-static-spheroid) present in the 3D-static-spheroid culture medium prepared in Example 1.2 were analyzed. As an experimental group, the 3D-static-spheroid culture medium prepared in Example 1.2 was used, and as a comparative group, a 3D mesenchymal stem cell spheroid-type cell aggregate (hereinafter referred to as 3D-dynamic-PEG spheroid) culture medium prepared by culturing mesenchymal stem cells for 5 days was used according to a dynamic 3D cell culture method disclosed in Registered Patent (Application No. 10-2016-0053026, method for producing stem cell-derived extracellular vesicles). With an optical microscope, microwells containing each culture medium were observed, which were illustrated in FIGS. 2A and 2B, and changes in spheroid-type cell aggregate area after culture compared to the initial culture was illustrated in FIG. 2C.

As illustrated in FIG. 2, as a result of comparing the areas of the 3D-static-spheroid of the experimental group and the 3D-dynamic-PEG spheroid of the comparative group, depending on a characteristic in which cells were densely condensed to form spheroids, compared to 3D-dynamic-PEG spheroids, 3D-static-spheroids showed a statistically significant decrease in area compared to spheroids at the beginning of culture. (p = 0.0011).

### Example 3. Size analysis of 3D spheroid-type cell aggregate

The size distributions of the 3D-static-spheroid prepared in Example 1.2 and the 3D-dynamic-PEG spheroid prepared in Example 3 were measured, and based on the measured size distribution data, dispersion coefficient, skewness and kurtosis were analyzed.

The skewness is a parameter capable of determining the inclined direction and degree of the distribution from the trend of a median value, and indicates a distribution with a longer tail to the right as closer to 1, and indicates a distribution with a longer tail to the left as closer to - 1. In the case of a normal distribution, the skewness is 0.

The kurtosis is a parameter that indicated the degree of sharpness of the data distribution, and if the value is positive, it means that a relatively large number of data points are accumulated in the central part, and if the value is negative, it means that relatively few data are accumulated in the central part. In the case of a normal distribution, the kurtosis is 0.

The measured size distribution data was illustrated in FIG. 3, and the results of analyzing the data was illustrated in Table 1.

**[Table 1]**

| | 3D-dynamic-PEG spheroid | 3D-static-spheroid |
|---|---|---|
| The number of spheroids | 154 | 155 |
| Size range | 108.4 - 225.0 um | 55.0 - 95.0 um |
| Size (mean ± SD) | 148.66 ± 20.89 um | 74.43 ± 7.756 um |
| Coefficient of variance | 14.1% | 9.59% |
| Skewness | 0.694 ± 0.195 | - 0.745 ± 0.195 |
| Kurtosis | 0.350 ± 0.389 | 1.799 ± 0.389 |

As illustrated in FIG. 3 and Table 1, it was confirmed that the average size of the 3D-static-spheroids was 74.43 um, and the coefficient of variance (CV) was 9.59%. In contrast, it was confirmed that the 3D-dynamic-PEG spheroids had an average size of 148.66 um and a coefficient of variance (CV) of 14.1%. To compare and test the measured coefficient of variance, as a result of performing a Feltz and Miller's (1996) asymptotic test, a p value was calculated as 0.01765604, and as a result of performing a Krishnamoorthy and Lee's (2014) modified signed-likelihood ratio test, the p value was calculated as 0.01853969. Accordingly, in both the tests, it was confirmed that the size distributions of 3D-static-spheroids and 3D-dynamic-PEG spheroids showed statistically significant different patterns.

As a result of comparing the size distributions of the 3D-static-spheroids and the 3D-dynamic-PEG spheroids, it was confirmed that the kurtosis value of the size distribution of the 3D-static-spheroids was relatively large, and the size distribution of the 3D-static-spheroids of the present invention showed a tendency to be concentrated in the median values. From these results, it was confirmed that when the 3D spheroids were produced by the method for producing the 3D-static-spheroids of the present invention, it was possible to produce 3D spheroids having a relatively constant size.

### Example 4. Morphological analysis of extracellular vesicles derived from 3D spheroid-type cell aggregate

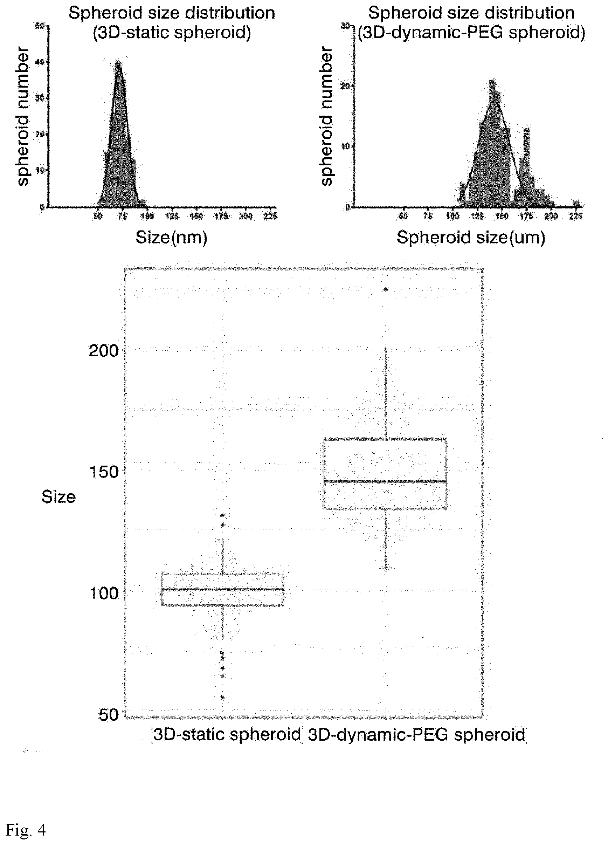

In order to observe the morphology of the 3D-static-spheroid EVs isolated in Example 1.3, the morphology was photographed using a transmission electron microscopy (TEM). Specifically, the 3D-static-spheroid EVs were fixed with 1% OsO₄ dissolved in a 0.1 M phosphate buffer (PB) for 2 hours. The EM grids were adsorbed onto the droplets of extracellular vesicles with a formvar side facing downward for 1 minute. Thereafter, the EM grids were blotted with filter paper and reacted with 2% uranyl acetate for 15 seconds. Excessive uranyl acetate was removed, and the EM grids were observed using a TEM (JEM-1011, JEOL, Japan), and the observed image was illustrated in FIG. 4.

As illustrated in FIG. 4, it was confirmed that the 3D-static-spheroid EV had a round shape, which was a typical shape of extracellular vesicles.

### Example 5. Concentration and size analysis of extracellular vesicles derived from 3D spheroid-type cell aggregate

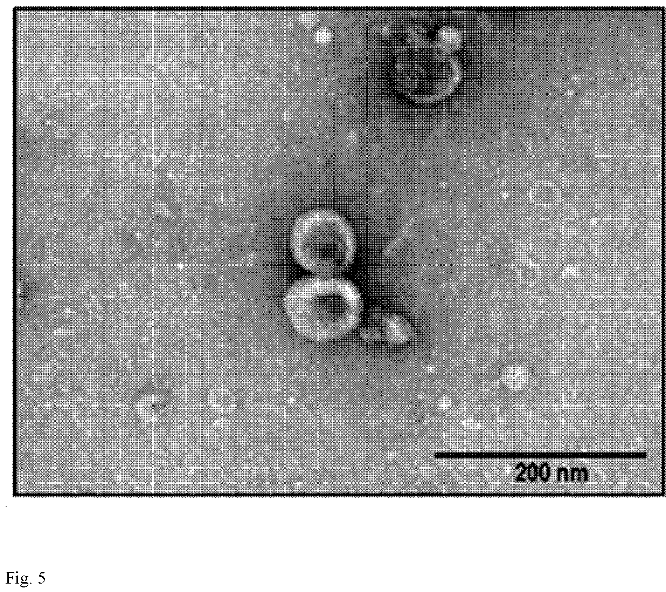

In order to confirm the concentration and size distribution of 3D-static-spheroid EVs isolated in Example 1.3, nanoparticle tracking analysis (NTA) was performed using NanoSight NS300 (Malvern, Worcestershire, UK). For optimal analysis, the 3D-static-spheroid EVs were pre-diluted in a vesicle-free phosphate buffer (PBS). The average size and concentration (particle/mL) were calculated by integrating three records, and the results were illustrated in FIG. 5.

As illustrated in FIG. 5, it was confirmed that the average particle diameter of the 3D-static-spheroid EV was 182.5 nm and the mode diameter was 106.1 nm.

### Example 6. Analysis of expression markers of extracellular vesicles derived from 3D spheroid-type cell aggregate

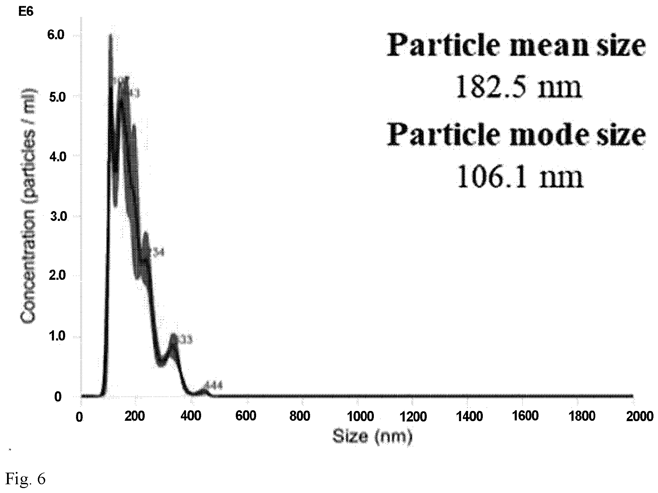

Experiments were performed to confirm expression markers of 3D-static-spheroid EVs isolated in Example 1.3. A cell lysate and a secretome were used as a control. The cell lysate was prepared by a method of washing 3D-static-spheroids with PBS, treating the 3D-static-spheroids with trypsin, restoring cells, and obtaining a cell pellet using a centrifuge of the restored cells. The secretome was prepared by a method of obtaining the cell pellet, isolating EVs from a supernatant of the culture medium through the process of Example 1.3, and obtaining the remaining culture secretome. For marker analysis, extracellular vesicles-specific positive markers CD9, CD63, CD81 and HSP70 were quantified using ELISA, and specific contaminant protein markers such as calreticulin, histone H2A.Z, cytochrome C, albumin, and antibiotics were quantified. In addition, histone H2A.Z, histone H3, lamin A/C, and calreticulin, which were specific contaminant protein markers of extracellular vesicles, were quantified using Western blot, and a positive marker of extracellular vesicles, flotillin-1 was quantified. The ELISA analysis result and the Western blot result were illustrated in FIG. 6.

As illustrated in FIG. 6, it was confirmed that 3D-static-spheroid EVs expressed all of the extracellular vesicles-specific positive markers CD9, CD63, CD81 and HSP70, and calreticulin, histone H2A.Z, histone H3, cytochrome C, albumin (BSA, bovine serum albumin), lamin A/C, and antibiotics, which were highly expressed contaminant protein markers in the cell lysate and the secretome, were not almost expressed. In particular, it was confirmed that flotillin-1, the positive marker for extracellular vesicles, which was expressed very low in the cell lysate, was relatively highly expressed in 3D-static-spheroid EVs.

### Example 7. Analysis of production of extracellular vesicles derived from 3D spheroid-type cell aggregate

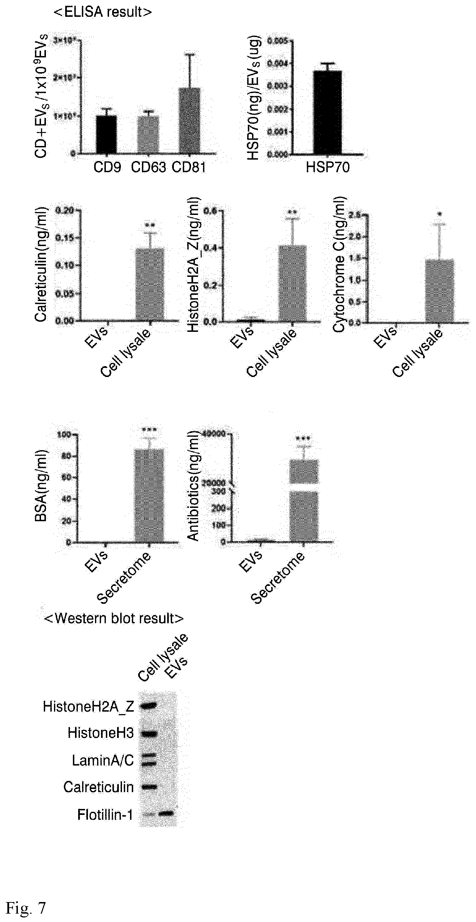

The experiment was performed to compare the productions of the 3D-static-spheroid EVs, which were the extracellular vesicles produced by the production method of Example 1, extracellular vesicles (3D-dynamic-PEG spheroid EVs) isolated from the 3D-dynamic-PEG spheroids of Example 2, and extracellular vesicles (2D-EVs) isolated from stem cells cultured by a conventional 2D culture method. The 2D-EVs were prepared by the following process. The stem cells cultured for 3 days in a cell stack were washed with PBS, replaced with a serum-free medium, and further cultured for 2 days. The culture medium was restored and the cell debris was continuously removed using centrifugation and a 0.2 µm filter. Thereafter, the culture medium passed through a hollow fiber membrane using a TFF system to remove proteins, and the EVs were first isolated, and purified once more with physiological saline to obtain high-purity 2D-EVs. The productions per cell derived from the prepared 3D-static-spheroid EVs, 3D-dynamic-PEG spheroid EVs, and 2D-EVs were compared and illustrated in Table 2 and FIG. 7.

**[Table 2]**

| | 2D-EV | 3D-dynamic-PEG spheroid EV | 3D-static-spheroid EV |
|---|---|---|---|
| Production (EVs/cell) | 2437 EVs/cell | 6791 EVs/cell | 6840 EVs/cell |

### Example 8. Expression microRNA (miRNA) analysis of extracellular vesicles derived from 3D spheroid-type cell aggregate

### 8.1 Identification of miRNAs with increased expression in 3D-static-spheroid EV

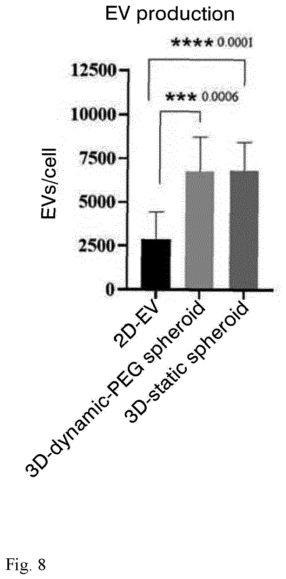

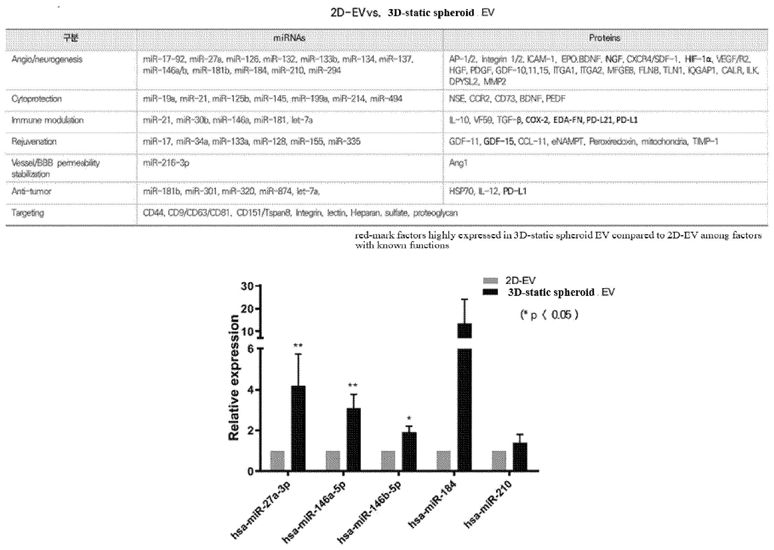

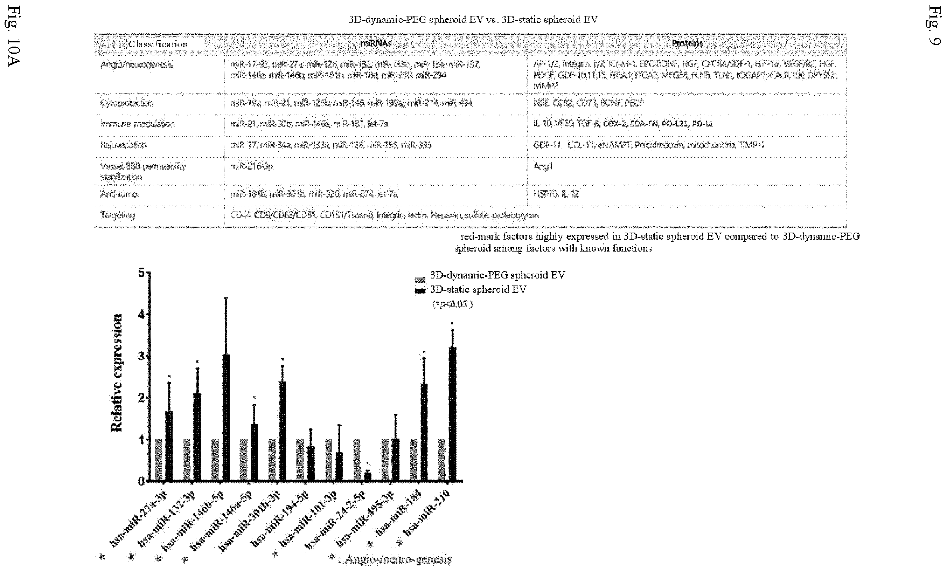

In order to confirm the differences in characteristics between 3D-static-spheroid EVs which were the extracellular vesicles produced by the production method of Example 1, 3D-dynamic-PEG spheroid EVs which were extracellular vesicles isolated from 3D-dynamic-PEG spheroids of Example 2, and 2D-EVs prepared in Example 7, expression of miRNAs and protein expression were measured by qPCR. miRNAs and proteins highly expressed in the 3D-static-spheroid EVs compared to the 2D-EVs were illustrated in FIG. 8, and miRNAs and proteins highly expressed in the 3D-static-spheroid EVs compared to the 3D-dynamic-PEG spheroid EVs were illustrated in FIG. 9.

As illustrated in FIG. 8, it was confirmed that as compared with the 2D-EVs, the 3D-static-spheroid EVs highly expressed miR-27a, miR-146b, and miR-146a as miRNAs that exhibited the efficacy on angio/neurogenesis and immune regulation, and highly expressed integrin 1/2 and Vascular endothelial growth factor/R2 (VEGF/R2) as proteins exhibiting the efficacy on angio/neurogenesis.

As illustrated in FIG. 9, it was confirmed that as compared with the 3D-dynamic-PEG spheroid EVs, the 3D-static-spheroid EVs highly expressed miR-146a as miRNA exhibiting the efficacy on angio/neurogenesis and immune regulation, miR-27a, miR-132, miR-184 and miR-210, which were miRNAs exhibiting the efficacy on angio/neurogenesis, and miR-301b exhibiting antitumor efficacy, and highly expressed integrin 1/2 and VEGF/R2 as proteins exhibiting the efficacy on angio/neurogenesis.

From these results, it was confirmed that as compared to the 2D-EVs or 3D-dynamic-PEG spheroid EVs, the 3D-static-spheroid EVs, which were the extracellular vesicles produced by the production method of Example 1, were new extracellular vesicles with different miRNA and protein expression, and particularly, extracellular vesicles which may be clinically useful by highly expressing miRNAs related to angio/neurogenesis, immune regulation, rejuvenation or antitumor.

### 8.2 Analysis of miRNA expression difference according to donor

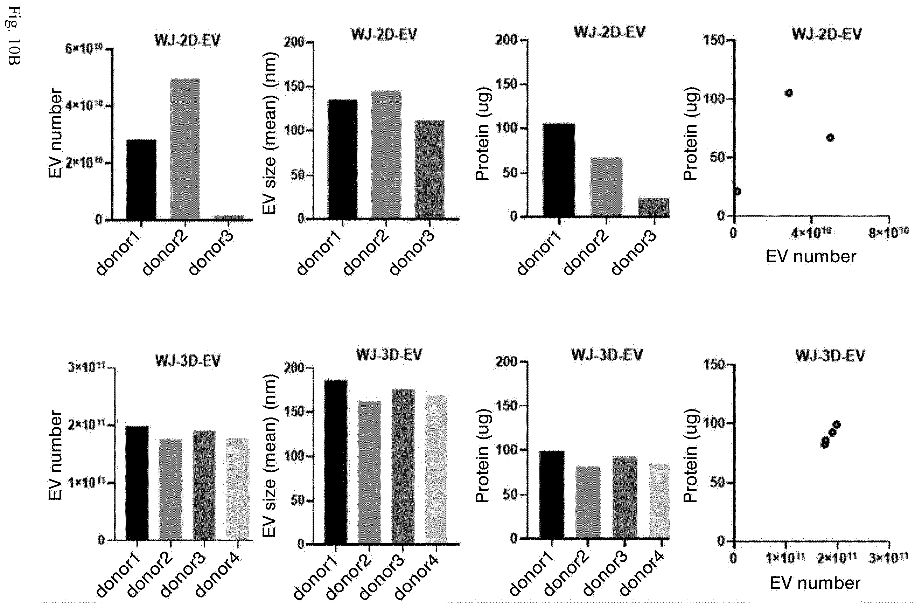

Stem cell therapy was known to have a problem of donor variation in which components varied depending on a donor. An experiment was performed to confirm whether the 3D-static spheroid EVs of the present invention exhibited a more consistent miRNA profile without a donor variation issue. Samples from each donor were received from the Samsung medical center (Seoul, Korea) and used. Donor-specific miRNA profiles were compared with each other in 2D-cultured WJ-MSCs and 3D-cultured WJ-MSCs of Example 1.1 and 3D-static-spheroid EVs, which were the extracellular vesicles produced by the production method of Example 1. miRNA was profiled by a Small RNA sequencing method, and as a result, the numbers of EVs produced by each donor, EV protein amounts, and miRNA profiles were compared with each other in 2D-cultured WJ-MSCs and 3D-cultured WJ-MSCs of Example 1.1 and 3D-static-spheroid EVs, which were the extracellular vesicles produced by the production method of Example 1. miRNA was profiled by a Small RNA sequencing method, and the results were illustrated in FIG. 10A and FIG. 10B.

As illustrated in FIG. 10A, 2D-cultured WJ-MSC-derived 2D-EVs showed variations in the amount and size of EVs produced and amount of EV-produced proteins depending on each donor, but the 3D-static-spheroid EVs showed consistent results without a large variation between donors.

In addition, as illustrated in FIG. 10B, it was confirmed that 2D-cultured WJ-MSCs and 2D-EVs had a large difference in miRNA composition produced depending on a donor, whereas 3D-cultured WJ-MSCs and 3D-static-spheroid EVs had a decreased difference depending on a donor. In particular, it was confirmed that the 3D-static-spheroid EVs showed a consistent miRNA profile without a difference depending on a donor, and at the same time, as compared with 2D-EVs, miR-27a-3p (1.5-fold), miR-146a-5p (2.3-fold), miR-210 (2.6-fold), and miR-132 (2.6-fold), which were miRNAs capable of exhibiting the angiogenesis effect, were more uniformly included. In addition, it was confirmed that miR-199a, miR-125b, miR-26a, let-7, miR-125a, miR-181b, and miR-92a, which were miRNAs related to angiogenesis, were expressed at uniform levels among respective donors.

These results indicate that the 3D-static-spheroid EVs may reduce a difference in therapeutic active ingredients according to a donor and exhibit better therapeutic effects.

### Example 9. Analysis of expression mRNA when treating cells with extracellular vesicles derived from 3D spheroid-type cell aggregate

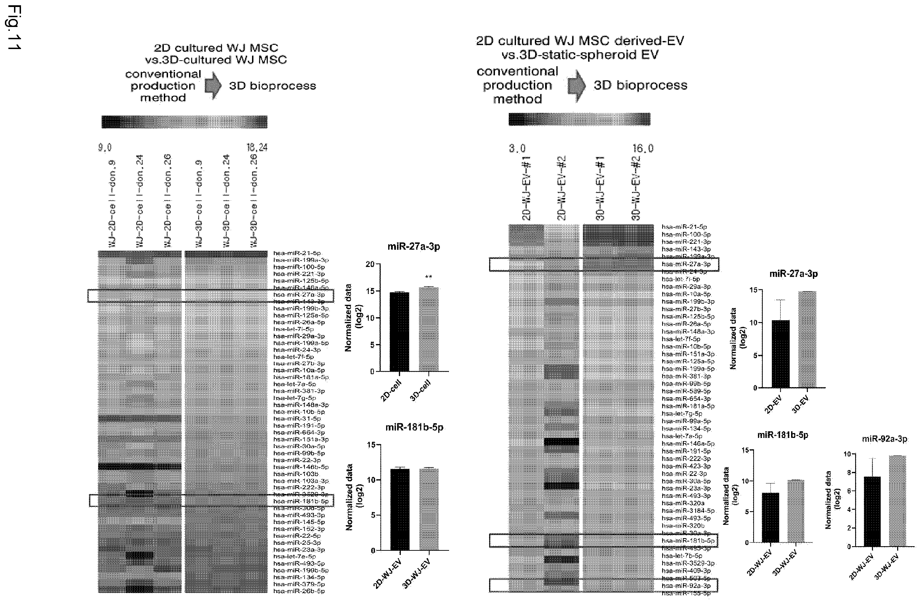

Human umbilical vascular endothelial cells (HUVECs) or primitive neural stem cells (pNSCs) were treated with 3D-static-spheroid EVs, which were the extracellular vesicles produced by the production method of Example 1, and 2D-EVs, which were extracellular vesicles isolated from stem cells cultured by a conventional 2D culture method, and then changes in mRNA expression were confirmed through qPCR, and the qPCR results were illustrated in FIG. 11.

As illustrated in FIG. 11, compared to cells treated with 2D-EVs, the HUVECs treated with the 3D-static-spheroid EVs increased the expression of a vascular endothelial growth factor (VEGF), a hypoxia-inducible factor 1-alpha (Hif-1a), and a fibroblast growth factor (FGF), and the pNSCs treated with the 3D-static-spheroid EVs increased the expression of VEGF, a brain-derived neurotrophic factor (BDNF), FGF, a nerve growth factor (NGF), and a hepatocyte growth factor (HGF). In particular, it was confirmed that in the case of pNSCs treated with the 2D-EVs compared to a control group, the expression of NGF and HGF decreased, but pNSCs treated with the 3D-static-spheroid EVs showed an effect of increasing the expression of NGF and HGF, and thus the 3D-static-spheroid EVs and 2D-EVs exhibited qualitatively different effects.

The VEGF, BDNF, NGF, HGF and Hif-1a were angio/neurogenesis related proteins, and the FGF was a factor corresponding to a protein that regulated biological functions related to cell proliferation, survival, and differentiation. Thus, it was confirmed that 3D-static-spheroid EVs with relatively high increased expression effects of VEGF, BDNF, NGF, HGF, Hif-1a and FGF were excellent extracellular vesicles for clinical application.

### Example 10. Confirmation of wound healing effect in animal model of extracellular vesicles derived from 3D spheroid-type cell aggregate

Through Example 8, it was confirmed that 3D-static-spheroid EVs, which were extracellular vesicles prepared by the preparation method of Example 1, were new extracellular vesicles with different expression patterns of miRNA and protein from conventional 2D-EV or 3D-dynamic-PEG spheroid EVs.

To determine whether 3D-static-spheroid EVs, which were the newly prepared extracellular vesicles, exhibited a wound healing effect, an animal wound model was prepared and an experiment was performed as follows to confirm a wound recovery effect.

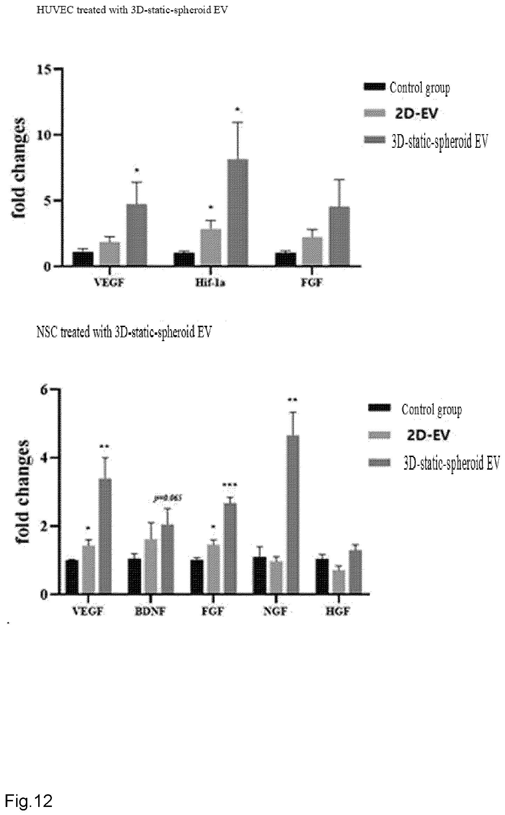

After anesthetizing a 8-week SD rat, the hair on the back was removed, and the skin on the back was folded along the midline of the back, and punched using an 8 mm skin biopsy punch to prepare a wound model. A total of 6 × 10⁸/100 µl of 3D-static-spheroid EVs were injected subcutaneously into four points around the wound, and a control group was injected with an equal volume of 100 µl of PBS. Additionally, to compare the wound healing effects of 3D-static-spheroid EV and WJ-MSC, the WJ-MSC administered group was set as a comparative experimental group. After making a skin wound on the back of the rat, 2 ×x 10⁶ Cells/100 µl of WJ-MSCs were injected intradermally into four points around the wound, and then observed for 2 weeks. The injection was administered once a day for three days, and a silicone pad punched with a diameter of 10 mm was sewn and fixed onto the wound with nylon 6.0 to prevent the wound from shrinking. The results of observing the wound recovery effect after injection were illustrated in FIG. 12.

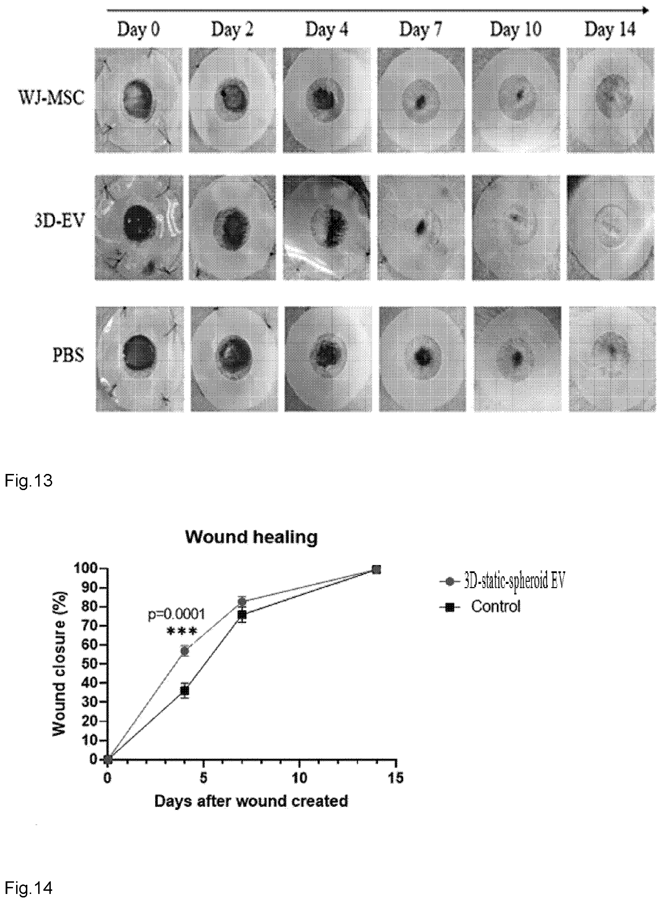

As illustrated in FIG. 12, the 3D-static-spheroid EV treated group showed a faster wound recovery effect compared to the control and WJ-MSC treated groups. A difference in wound recovery effect between the 3D-static-spheroid EV and the control group was quantified and illustrated in FIG. 13, and it was confirmed that the 3D-static-spheroid EV treated group showed a significantly faster wound recovery effect than the control group from day 4 after the start of treatment.

The wound recovery effect of 3D-static-spheroid EVs was confirmed through a hematoxylin and eosin (H&E) staining experiment on the skin cross-sectional area. After previously prepared rat wound modeling, the cross-sectional area of the wounded skin was stained on days 3, 7, and 14 of 3D-static-spheroid EV treatment, and the thickness of the epidermal portion of the skin was measured 14 days after treatment.

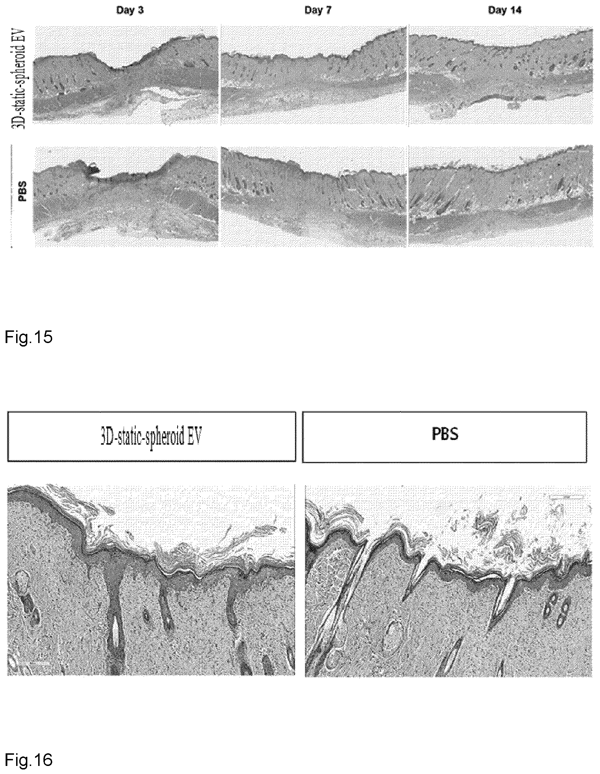

As illustrated in FIG. 14, it was confirmed that the skin was rapidly generated in the 3D-static-spheroid EV treated group, and as illustrated in FIG. 15, it was confirmed that the epidermal thickness was significantly increased in the 3D-static-spheroid EV treated group after 14 days of treatment.

More specifically, the thicknesses of the epidermis and the epidermal periphery were measured and quantified, which were illustrated in FIG. 16.

As illustrated in FIG. 16, it was confirmed that 3D-static-spheroid EVs exhibited a rapid skin thickness increasing effect in the epidermis and the epidermal periphery, which could rapidly recover the wound.

### Example 11. Wound healing effect in chamber model of extracellular vesicles derived from 3D spheroid-type cell aggregate

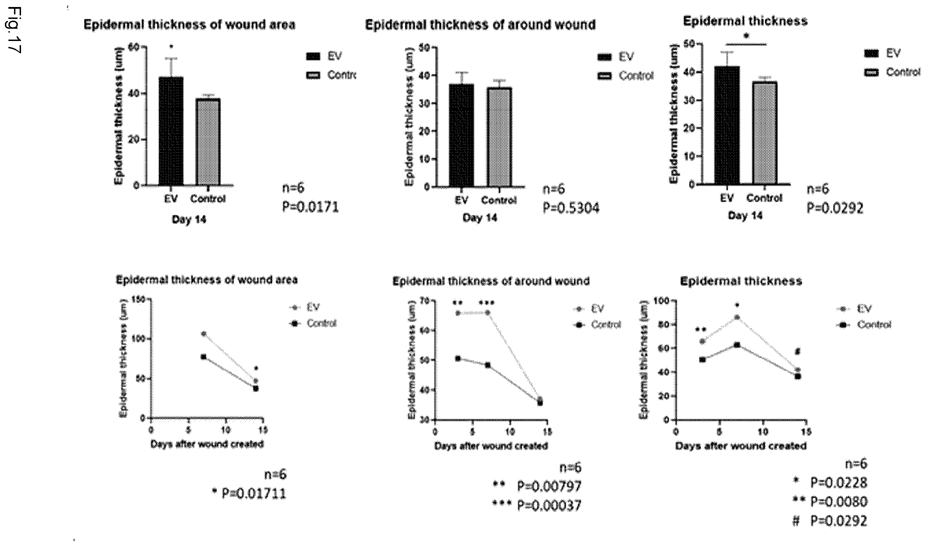

A wound healing effect of extracellular vesicles derived from a 3D spheroid-shaped cell aggregate was confirmed in a chamber model, and rat skin wound modeling was performed, and the experimental method was illustrated in FIG. 17.

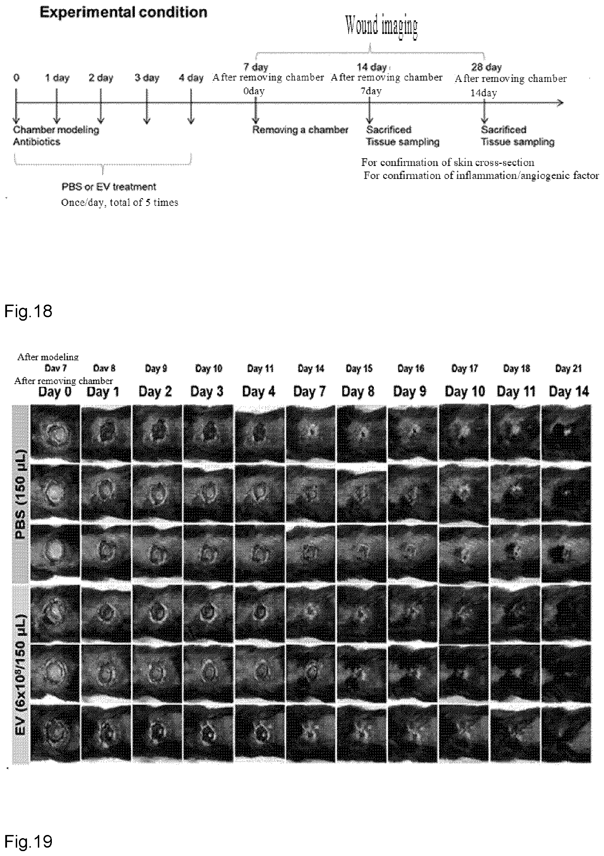

The result of daily photographing the wound recovery effect after removing the chamber was illustrated in FIG. 18, and a change in size of the wound area was quantified and illustrated in FIG. 19.

As illustrated in FIGS. 18 and 19, a significant difference in the wound recovery area was confirmed at 3 and 10 days after removing the chamber, and it was confirmed that the wound recovery rate after removing the chamber was faster in the group treated with the extracellular vesicles derived from the 3D spheroid-shaped cell aggregate.

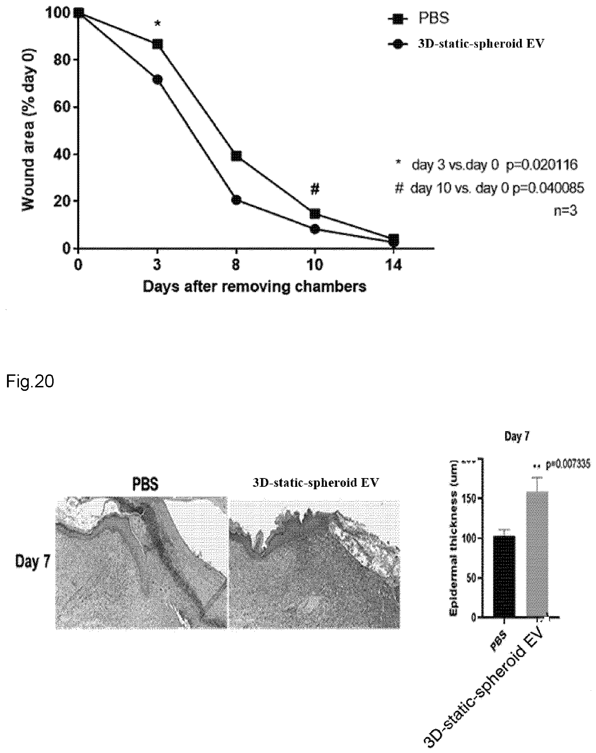

The increase in epidermal thickness in the chamber model was confirmed through the hematoxylin and eosin (H&E) staining experiment, and the results were illustrated in FIG. 20.

As illustrated in FIG. 20, it was confirmed that the epidermal layer thickness was significantly increased in the group treated with the extracellular vesicles derived from the 3D spheroid-shaped cell aggregate at 7 days after removing the chamber compared to PBS, and then the epidermal thickness was maintained at a similar level on 14 day.

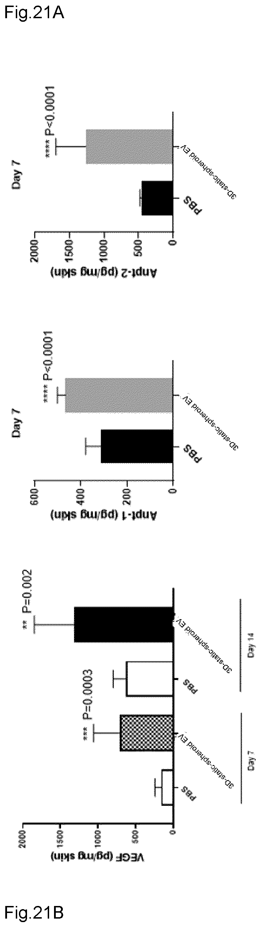

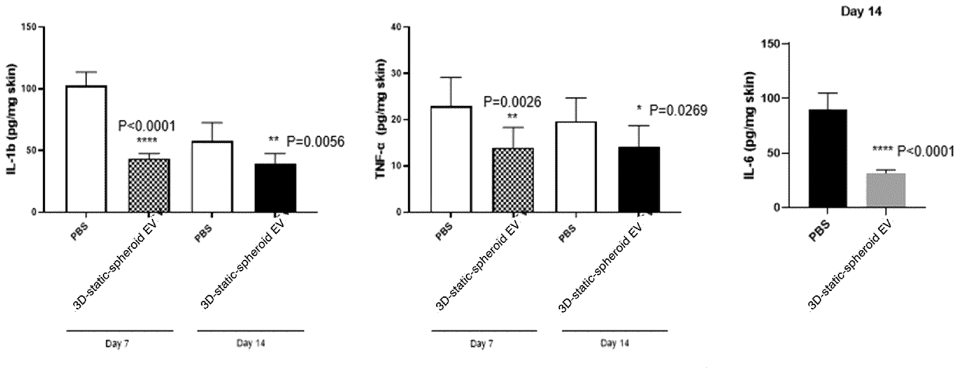

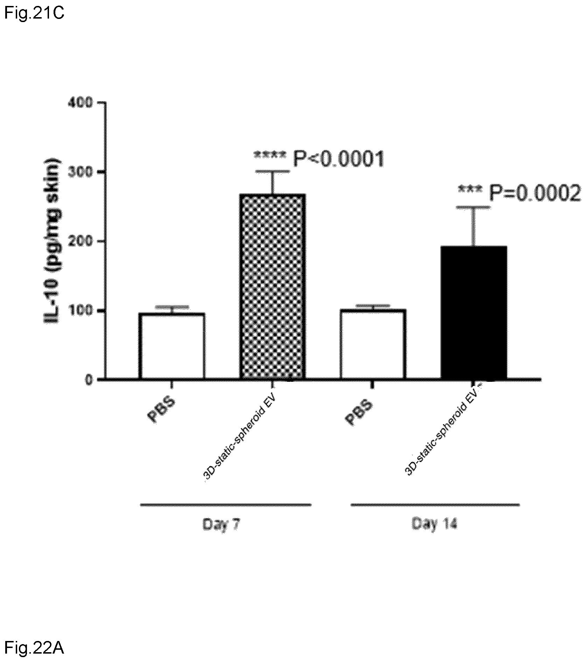

In addition, changes in expression of VEGF, angiopoietin-2 (Angpt-2), IL-1b, and IL-10 were confirmed using ELISA at 7 or 14 days after removing the chamber in the chamber model, and the results were illustrated in FIG. 21.

As illustrated in FIG. 21, it was confirmed that in the group treated with the extracellular vesicles derived from the 3D spheroid-shaped cell aggregate, the expression of VEGF, angiopoietin-1 (Angpt-1), and angiopoietin-2 (Angpt-2), which were involved in vascular regeneration, was increased at 7 and 14 days after removing the chamber, the expression of IL-10 involved in anti-inflammation was increased, and the expression of IL-1b, TNF-a, and IL-6, which were involved in inflammation, was decreased.

### Example 11. Confirmation of cell motility according to group treated with extracellular vesicles derived from 3D spheroid-shaped cell aggregate

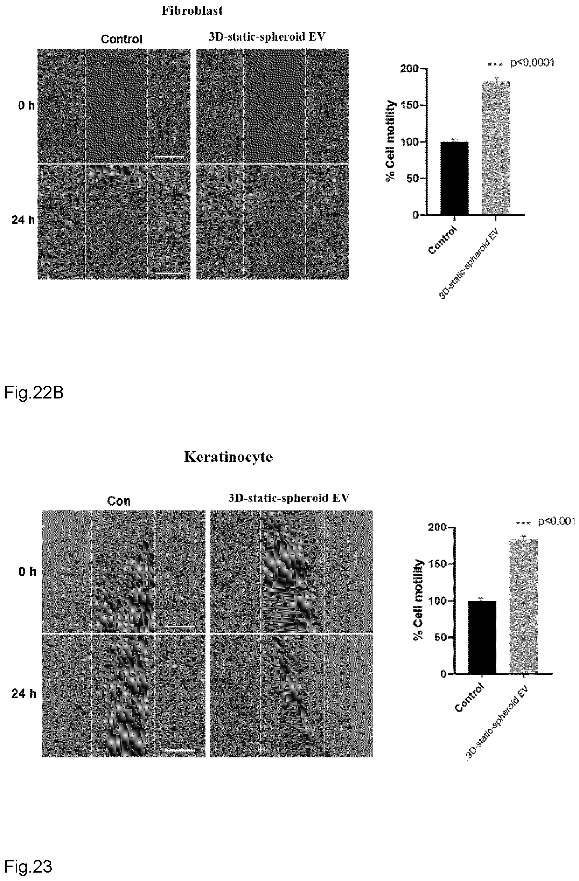

A scratch model was prepared, and the mobility of fibroblasts and keratinocytes after treatment with the extracellular vesicles derived from the 3D spheroid-shaped cell aggregate of the present invention was confirmed, and the results were illustrated in FIG. 22.

As illustrated in FIGS. 22A and 22B, it was confirmed that in a group treated with a 3D spheroid-shaped cell aggregate, the mobility of fibroblasts and keratinocytes was significantly increased, and as a result, rapid wound recovery could be achieved.

### Example 12. Confirmation of angiogenesis promoting effect of extracellular vesicles derived from 3D spheroid-type cell aggregate

After vascular endothelial cells (HUVECs) were treated with 3D-static-spheroid EVs as the extracellular vesicles prepared by the preparation method of Example 1, and 2D-EVs as extracellular vesicles isolated from stem cells cultured by a conventional 2D culture method, changes in expression of angiogenesis-related factors, such as a vascular endothelial growth factor (VEGF), a hypoxia-inducible factor 1-alpha (Hif-1a) and a fibroblast growth factor (FGF), were confirmed, and the results were illustrated in FIG. 23.

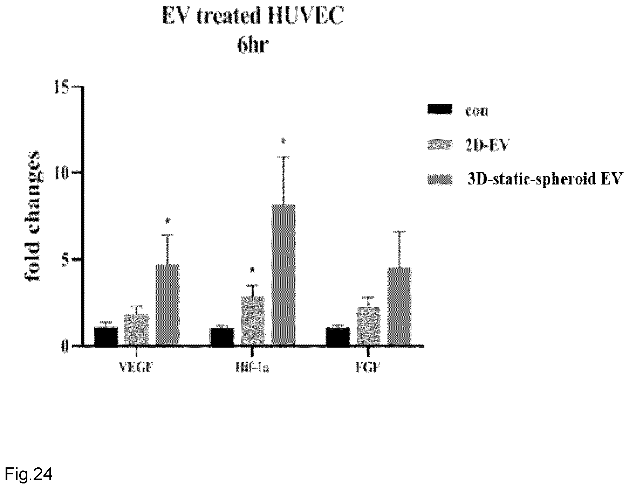

In addition, in order to confirm an effect of proliferation of neovascularization by extracellular vesicles obtained through a 3D stem cell culture, HUVECs attached on Matrigel were treated with WJ-2D-EV and 3D-static-spheroid EV of the present invention, and then a tube formation effect was confirmed. Specifically, HUVECs were cultured in an M199 medium (Gibco) supplemented with 20% FBS, 5 U/mL heparin, and 3 ng/mL bFGF. The cells were inoculated on a growth factor-reduced Matrigel Matrix (BD Bioscience, MA, USA) from µ-Slides Angiogenesis (ibidi, Graefelfing, Germany) at a density of 1.7 × 10⁴ cells, and the tube was formed for 4 hours in a humidifying chamber under a 37°C and 5% CO₂ environment. Images were taken on a phase-contrast microscope (Olympus), and the number of tubular structures was quantified in a microscope field (4× magnification) using ImageJ software, and the results were illustrated in FIG. 24.

As illustrated in FIG. 23, it was confirmed that compared to cells treated with 2D-EVs, in the vascular endothelial cells (HUVECs) treated with 3D-static-spheroid EVs, the expression of a vascular endothelial growth factor (VEGF), a hypoxia-inducible factor 1-alpha (Hif-1a) and a fibroblast growth factor (FGF) was increased. The VEGF and Hif-1a were angiogenesis-related proteins, and the FGF was a factor corresponding to a protein that regulated biological functions related to cell proliferation, survival, and differentiation, and the 3D-static-spheroid EVs were excellent clinical application in angiogenesis.

As can be seen in FIG. 24, it was confirmed that in the experimental group treated with the 3D-static-spheroid EV of the present invention, tube formation was significantly increased compared to a group treated with VEGF, an angiogenesis-related protein, and had an excellent tube formation effect even compared to the WJ-2D-EV treated group.

### Example 13. Verification of 3D-static-spheroid EV effect according to preparation conditions

### 13.1 Experimental methods and conditions

Through Examples above, the excellent effect of the 3D-static-spheroid EVs of the present invention was confirmed. The preparation method of Example 1 was performed in the same manner, but to confirm whether the same effect was observed even in EVs prepared under different microwell specifications and cell number conditions, the cells were incubated by changing the cell count standard per microwell from 400 cells/well to 200 cells/well, or changing the microwell specification from 500 µm × 200 µm in diameter and depth to 500 µm × 600 µm, or a flat well with a diameter of 800 µm and no depth.

The experimental conditions changed from the preparation method of Example 1 were shown in Table 3 below.

**[Table 3]**

| | Experimental Example 1 | Experimental Example 2 | Experimental Example 3 |
|---|---|---|---|
| Number of cells per microwell (cell/microwell) | 400 | 400 | 200 |
| Diameter of microwell (µm) | 500 | 800 | 4-500 |
| Depth of microwell (µm) | 600 | / | 200 |

The WJ-MSCs of passage 6 stage prepared in Example 1.1 were washed with PBS, treated with trypsin (TrypLETM Express, GIBCO, NY, USA) and reacted in a CO₂ incubator for 5 minutes. Thereafter, a new serum-free medium was added to neutralize trypsin and the cells were restored to obtain a cell pellet using a centrifuge. Then, a new serum-free medium was added to prepare a cell suspension, and the cells were counted. After counting the cells, the cells were uniformly dispensed into microwells under the conditions shown in Table 3 above, maintained in a static state to induce spontaneous spheroid-type cell aggregate formation, and incubated in a CO2 incubator at 37°C for a total of 4 days to prepare a 3D spheroid-type cell aggregate culture medium.

### 13.2 Confirmation of 3D spheroid-type cell aggregate morphology

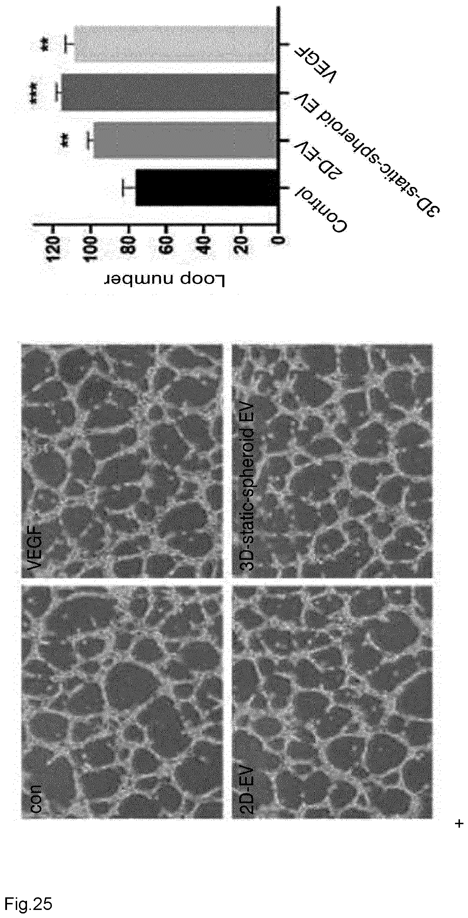

It was confirmed that spheroids were formed uniformly in the same manner as in Example 1 under the conditions of Experimental Examples 1 to 3, and the 3D spheroid-type cell aggregate culture medium was restored to obtain spheroid-derived extracellular vesicles by the method of Example 1.3. The size distribution of the obtained extracellular vesicles was measured, and the roundness and solidity of the spheroids were additionally confirmed, and the results were illustrated in FIG. 25.

As illustrated in FIG. 25, it was confirmed that the sizes of the 3D spheroid-shaped cell aggregates prepared in Experimental Examples 1 to 3 were within the range of 55 to 131 µm, which was the size range of the cell aggregate prepared in Example 1, and it was confirmed that the average values of the size distribution were 70.34 to 99.51 µm. In addition, as a result of confirming the roundness and solidity, it was confirmed that similarly to the average roundness value of 0.8697 (CV 2.64%) of the 3D spheroid-type cell aggregate of Example 1, the spheroid-type cell aggregates of Experimental Examples 1 to 3 also had the Roundness values of 0.8751, 0.8669, and 0.8601, respectively. In addition, it was confirmed that similarly to the average solidity value of 0.9488 (CV 2.64%) of the 3D spheroid-type cell aggregate of Example 1, the spheroid-type cell aggregates of Experimental Examples 1 to 3 also had the solidity values of 0.9744, 1, and 0.9752, respectively.

These results showed that even if the number of cells per microwell was changed to 200 or 400 and the diameter of the microwell was changed to 400 to 800, the 3D spheroid-type cell aggregate with a similar shape may be effectively formed through the method of Example 1.

### 13.3 Comparison of miRNA expression patterns of 3D spheroid-type derived EVs

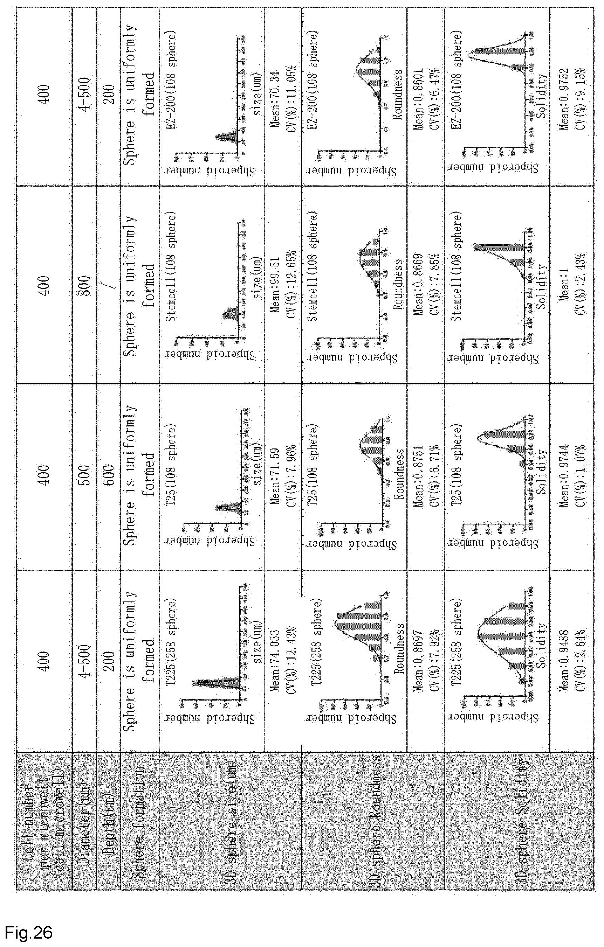

Since it was confirmed that spheroid-type cell aggregates having a similar shape to Example 1 could be prepared even under the conditions of Experimental Examples 1 to 3, additionally, extracellular vesicles were isolated and obtained from the spheroid-type cell aggregates by the method of Example 1.3, and it was confirmed whether the isolated and obtained EVs also showed the same miRNA expression patterns. Comparison of the miRNA expression patterns was confirmed using extracellular vesicles derived from the spheroid-type cell aggregates prepared by the methods of Experimental Examples 1 and 2 under different microwell conditions. Analysis of expressed miRNA was confirmed through the same qPCR method as in Example 8. The results were illustrated in FIG. 26. The miRNA expression pattern was compared with that of the 2D-EV prepared in Example 7.

As illustrated in FIG. 26, it was confirmed that even if the microwell conditions were changed to 500 and 800 µm in diameter, in the same manner as the EVs prepared in Example 1, the expression of miR-132 and miR-210, which were miRNAs exhibiting the efficacy on angio/neurogenesis and immune regulation, was significantly increased compared to the existing 2D-EVs. These results showed that EVs derived from the three-dimensional spheroid-type cell aggregates obtained by the method of the present invention using microwells with a diameter of 200 to 800 µm may commonly exhibit miRNA marker expression characteristics. Therefore, the EVs may all be referred to as 3D-static-spheroid EVs.

### 13.4 Confirmation of angiogenesis effect of 3D spheroid-derived EVs

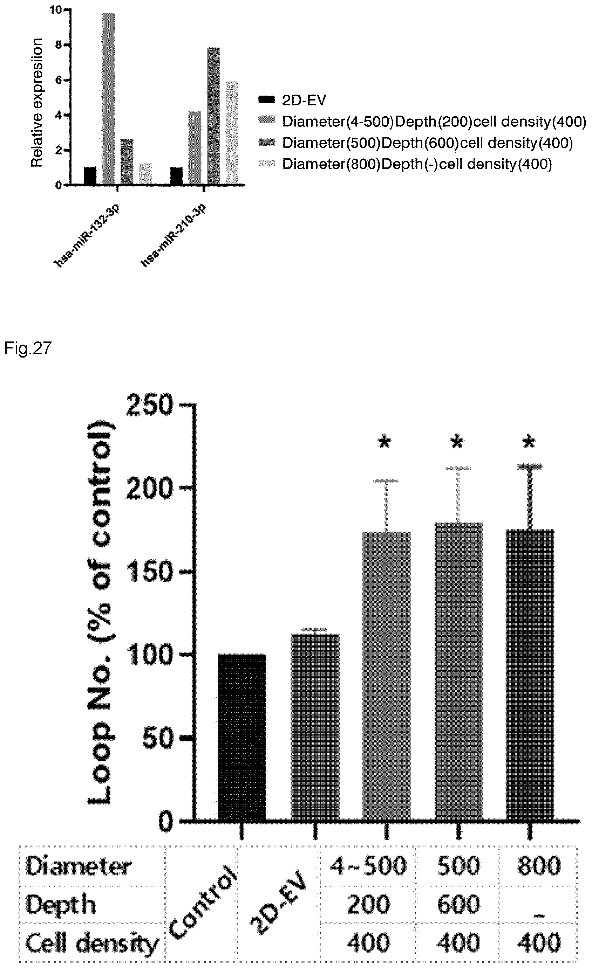

An experiment was performed to confirm the angiogenesis effect of 3D-static-spheroid EVs obtained through the method of the present invention using microwells with a diameter of 200 to 800 µm. As experimental groups, 3D-static-spheroid EVs prepared under the conditions of Experimental Examples 1 and 2 and Example 1, and 2D-EVs prepared in Example 7 were used, and the tube formation effect was confirmed in the same manner as in Example 11. The results were illustrated in FIG. 27.

As illustrated in FIG. 27, all 3D-static-spheroid EVs prepared under the conditions of Experimental Examples 1 and 2 and Example 1 showed a significantly excellent tube formation effect compared to the control group and 2D-EV.

Based on the results, it was confirmed that the 3D-static-spheroid EVs had an excellent effect in promoting angiogenesis, and through this, the 3D-static-spheroid EVs may achieve excellent wound recovery compared to other EVs such as WJ-2D-EVs.

As described above, specific parts of the present invention have been described in detail, and it will be apparent to those skilled in the art that these specific techniques are merely preferred embodiments, and the scope of the present invention is not limited thereto. Therefore, the substantial scope of the present invention will be defined by the appended claims and their equivalents.

## Claims

1. A pharmaceutical composition for use in wound healing or wound recovery comprising extracellular vesicles derived from a three-dimensional spheroid-type cell aggregate prepared by (a) preparing a three-dimensional spheroid-type cell aggregate by 3-dimensionally (3D) culturing stem cells in a microwell with a diameter of 200 to 800 µm and a depth of 100 to 1000 µm; and (b) isolating extracellular vesicles from the three-dimensional spheroid-type cell aggregate.

2. The pharmaceutical composition for use in wound healing or wound recovery of claim 1, wherein the stem cells are at least one selected from the group consisting of mesenchymal stem cells, pluripotent stem cells, induced pluripotent stem cells, and embryonic stem cells.

3. The pharmaceutical composition for use in wound healing or wound recovery of claim 1, wherein the 3D culture in step (a) is a static culture.

4. The pharmaceutical composition for use in wound healing or wound recovery of claim 1, wherein the 3D culture in step (a) is culturing mesenchymal stem cells dispensed into microwells at a density of 100 to 1,000 cells/well.

5. The pharmaceutical composition for use in wound healing or wound recovery of claim 1, wherein the extracellular vesicles highly express at least one selected from the group consisting of miR-146a, miR-27a, miR-132, miR-184, miR-210, and miR-301b compared to extracellular vesicles derived from a spheroid-type cell aggregate obtained by 3D dynamic culture of mesenchymal stem cells; or highly express at least one selected from the group consisting of miR-27a, miR-146a, and miR-146b compared to extracellular vesicles derived from 2D-cultured mesenchymal stem cells.

6. The pharmaceutical composition for use in wound healing or wound recovery of claim 1, wherein the extracellular vesicles promote the mobility of fibroblasts or keratinocytes at the wound area.

7. The pharmaceutical composition for use in wound healing or wound recovery of claim 1, wherein the extracellular vesicles promote angiogenesis at the wound area.

8. A cosmetic composition for use in wound healing or wound recovery comprising extracellular vesicles derived from a three-dimensional spheroid-type cell aggregate prepared by (a) preparing a three-dimensional spheroid-type cell aggregate by 3-dimensionally (3D) culturing stem cells in a microwell with a diameter of 200 to 800 µm and a depth of 100 to 1000 µm; and
(b) isolating extracellular vesicles from the three-dimensional spheroid-type cell aggregate.

9. A quasi-drug composition for use in wound healing or wound recovery comprising extracellular vesicles derived from a three-dimensional spheroid-type cell aggregate prepared by (a) preparing a three-dimensional spheroid-type cell aggregate by 3-dimensionally (3D) culturing stem cells in a microwell with a diameter of 200 to 800 µm and a depth of 100 to 1000 µm; and
(b) isolating extracellular vesicles from the three-dimensional spheroid-type cell aggregate.

10. A method for producing a composition for wound healing or wound recovery comprising extracellular vesicles derived from a three-dimensional spheroid-type cell aggregate comprising:
(a) preparing a three-dimensional spheroid-type cell aggregate by 3-dimensionally (3D) culturing stem cells in a microwell with a diameter of 200 to 800 µm and a depth of 100 to 1000 µm; and
(b) isolating extracellular vesicles from the three-dimensional spheroid-type cell aggregate.

11. A method for wound healing comprising:
(a) preparing a three-dimensional spheroid-type cell aggregate by 3-dimensionally (3D) culturing stem cells in a microwell with a diameter of 200 to 800 µm and a depth of 100 to 1000 µm;
(b) preparing extracellular vesicles derived from the three-dimensional spheroid-type cell aggregate by isolating the extracellular vesicles from the three-dimensional spheroid-type cell aggregate; and
(c) treating the extracellular vesicles derived from the three-dimensional spheroid-type cell aggregate prepared in step (b) to a subject in need thereof.
